**Europäisches Patentamt**

**European Patent Office**

**Office européen des brevets**

(19)

(11) Publication number: **0 206 632**
A1

## (12) EUROPEAN PATENT APPLICATION

(21) Application number: **86304427.7**

(22) Date of filing: **10.06.86**

(51) Int. Cl.⁴: **C 07 C 121/18, C 07 C 120/00**

(30) Priority: **10.06.85 GB 8514612**

(43) Date of publication of application: **30.12.86**
**Bulletin 86/52**

(84) Designated Contracting States: **GB**

(71) Applicant: **INDIAN EXPLOSIVES LIMITED, ICI House 34 Chowringhee Road, Calcutta-700 071 West Bengal (IN)**

(72) Inventor: **Rajaram, Potaraju, AI. R. C. Private Limited CAFI Site, Belapur Road Thane 400 601 Maharashtra (IN)**
Inventor: **Joshi, Milind Vishnu, AI. R. C. Private Limited CAFI Site, Belapur Road Thane 400 601 Maharashtra (IN)**

(74) Representative: **Collier, Jeremy Austin Grey et al, J.A.Kemp & Co. 14, South Square Gray's Inn, London WC1R 5EU (GB)**

(54) **Process for the production of acetonitrile.**

(57) Acetonitrile is made by reaction of ethanol with ammonia in the vapour phase at a temperature of 300 to 500°C in the presence of a catalyst comprising a molybdenum heteropoly-acid preferably on a silica support.

EP 0 206 632 A1

ACTORUM AG

PROCESS FOR THE PRODUCTION OF ACETONITRILE

The present invention relates to the production of acetonitrile by reaction of ethanol with ammonia.

Acetonitrile is produced on a bulk scale as the principal by-product in the production of acrylonitrile by the vapour phase ammoxidation of propylene. Unfortunately, among the other by-products which result from this reaction are carbon dioxide and hydrogen cyanide, the latter being extremely toxic. The multiplicity of products resulting from the ammoxidation of propylene in itself constitutes a deterrent towards the isolation from the mixture of any one or more of such products. Added to this, there is the hazard presented by the presence of hydrogen cyanide the toxicity of which renders it very difficult to work with. As a result, there have been several efforts to develop a simpler, more economical method for the direct synthesis of acetonitrile from other starting materials.

The present invention provides a process which overcomes or at least reduces the drawbacks in known methods for acetonitrile production, and makes it possible to produce acetonitrile in good yield.

According to the present invention, a process for the production of acetonitrile comprises reacting ethanol and ammonia in the vapour phase in the presence of a catalyst comprising a heteropolyacid of molybdenum at a temperature of from 300°C to 500°C and recovering the acetonitrile produced. Small quantities of 2-picoline, 4-picoline and ethylamine which may simultaneously be formed as impurities are removed during the recovery.

Temperature is a critical parameter in the process of the invention and by regulating the temperature of the reaction, it is possible to optimise the yield of acetonitrile. The preferred temperature range is from 350°C to 450°C, more especially from 380°C to 420°C.

Another important parameter which governs the high yield of acetonitrile is the feed molar ratio. According to a further feature, it has been found desirable to carry out the process of the invention with an excess of ammonia, the molar ratio of ethanol to ammonia being maintained between 1:3 and 1:6, more preferably from 1:3 to 1:4.

For greater efficiency of the reaction, ammonia and ethanol are first preheated, vapourised and mixed together before being passed over the catalyst.

To achieve the maximum yield of acetonitrile, the overall space velocity of the reactants is maintained at a gas hourly space velocity (GHSV) in the range of 5000-8000. Under these conditions, the exit products from the reactor have been found to contain acetonitrile with typically greater than 60% molar selectivity as determined by gas chromatography.

Acetonitrile may be separated from the reactor outlet mixture by fractional distillation.

The preferred molybdenum heteropolyacid catalyst is 12-molybdophosphoric acid having the formula of $H_3PMo_{12}O_{40}$.

The catalyst is preferably supported on a carrier, especially a silica carrier. Thus 12-molybdophosphoric acid may be dispersed on a carrier by conventional doping techniques. Such a dispersed catalyst makes possible a highly active transformation of ethanol and ammonia to acetonitrile.

A number of carriers, such as silica gel, kieselguhr, alumina or pumice, may be employed for supporting the dispersed catalyst. However, the use of silica gel as the carrier is essential for obtaining high yields of acetonitrile. Carriers such as kieselguhr, alumina and pumice reduce the selective formation of acetonitrile drastically and correspondingly increase unwanted by-product formation.

The silica gel used as the carrier is conventional adsorbent grade silica gel having a specific surface area of around 500 $m^2$/g and a bulk density of around 0.75 g/c.c. In practice, the carrier material is preferably loaded with from 10 to 35 weight per cent of the 12-molybdophosphoric acid catalyst. The loading of the catalyst on carrier is governed by practical considerations such as convenience and efficiency and the preferred loading is generally from about 20 to 30 weight per cent of the catalyst.

The process of the invention can be carried out conveniently at ambient pressure as a continuous process. A tubular reactor into which the catalyst is packed as a bed is generally preferred. The reactants, ethanol and ammonia, are preheated, vapourised and mixed and brought into contact with the preheated catalyst within the reactor. The precise contact or residence time may vary depending on the specific temperature employed and also in relation to the quantities of the reagents but a minimum contact time of about 1 second, preferably of about 5 seconds, is necessary. A contact time in excess of 20 seconds is seldom necessary.

The product mixture resulting from the reaction is collected, for example in a cold condenser, and acetonitrile is subsequently separated therefrom by any standard technique, e.g. by fractional distillation.

The invention is described in greater detail in the following Example:

EXAMPLE

Liquid ethanol in the form of a rectified spirit and ammonia gas were pumped at the rate of 0.05 ml/min. and 80 ml/min. respectively into a heated catalyst bed at 400°C through a series of preheaters, vapourisers and mixers. The catalyst bed consisted of 20% 12-molybdophosphoric acid (P:Mo::1:12) on silica gel. The products from the exit of the catalyst bed were passed into a receiver equipped with a dry ice trap to collect the volatile products.

Gas chromatographic analysis showed the product to consist of 62% acetonitrile, 22% 2- and 4-picolines, 12% ethylamines and 4% ethanol. Pure acetonitrile was separated from this mixture by fractional distillation.

- 5 -

## CLAIMS

1. A process for the production of acetonitrile which comprises reacting ethanol and ammonia in the vapour phase in the presence of a catalyst comprising a heteropolyacid of molybdenum at a temperature of from 300°C to 500°C and recovering the acetonitrile produced.

2. A process as claimed in claim 1 wherein said reaction is effected at a temperature of from 380°C to 420°C.

3. A process as claimed in claim 1 or 2 wherein said reaction is effected employing an excess ammonia in a molar ratio of ethanol to ammonia of from 1 : 3 to 1 : 6.

4. A process as claimed in claim 3 wherein said molar ratio of ethanol to ammonia is from 1 : 3 to 1 : 4.

5. A process as claimed in any of claims 1 to 4 wherein said ammonia and ethanol are first preheated, vapourised and mixed together before being passed over said catalyst.

6. A process as claimed in any of claims 1 to 5 wherein the overall space velocity of the reactants is maintained at a gas hourly space velocity in the range of 5000-8000.

7. A process as claimed in any of claims 1 to 6 wherein said heteropolyacid of molybdenum is 12-molybdo-phosphoric acid having the formula $H_3PMo_{12}O_{40}$.

8. A process as claimed in any of claims 1 to 7 wherein said catalyst is supported or dispersed on a carrier.

9.  A process as claimed in claim 8 wherein said carrier is silica gel.

10.  A process as claimed in any of claims 1 to 9 wherein the contact time of the reagents with the catalyst is from 1 to 20 seconds.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US-A-3 106 574 (J.L. CALLAHAN) * Claims; example I * | 1-10 | C 07 C 121/18 C 07 C 120/00 |
| A | US-A-2 487 299 (R.B. BISHOP) * Claims; column 3, lines 2-10,44-50 * | 1-10 | |
| A | FR-A-2·316 218 (MONSANTO) * Claims; page 24, lines 32,33 * | 1-10 | |

**TECHNICAL FIELDS SEARCHED (Int. Cl.4)**

C 07 C 120/00
C 07 C 121/00

The present search report has been drawn up for all claims

| Place of search | Date of completion of the search | Examiner |
|---|---|---|
| THE HAGUE | 19-09-1986 | WRIGHT M.W. |